# EUROPEAN PATENT APPLICATION

(11) **EP 2 172 244 A2**
(43) Date of publication of application: **07.04.2010**
(21) Application number: 09171242.2
(22) Date of filing: 24.09.2009
(51) Int. Cl.: A61N 1/05, A61B 5/0408, A61L 15/58

(54) **Biomedical electrode and method of formation thereof**

(30) Priority: 25.09.2008 US 237803
(71) Applicant: Tyco Healthcare Group LP, Mansfield, MA 02048 (US)
(72) Inventor: Copp-Howland, Warren W., Chicopee, MA 01013 (US)
(74) Representative: Gray, James

(57) **Abstract**

The present disclosure relates to biomedical electrodes incorporating hydrophobic material and methods of formation thereof.

## Description

### BACKGROUND

### 1. Technical Field

The present disclosure relates generally to biomedical electrodes and, in particular, to biomedical electrodes incorporating hydrophobic material and methods of formation thereof.

### 2. Background of Related Art

Biomedical electrodes are used to transmit electrical signals or currents between the body of a patient and external or remote equipment, such as diagnostic, therapeutic and/or monitoring devices. Such electrodes typically include a conductive composition or hydrogel adherable to or otherwise contactable with, the skin of the patient, and a conductor, which is electrically connected to the conductive composition and to the external equipment. Conventional biomedical electrodes include ECG (electrocardiograph) monitoring electrodes, TENS (transcutaneous electrical nerve stimulation) electrodes, and defibrillation electrodes. Electrodes are also used as electrosurgical dispersive pads and in wound treatment applications.

Common problems associated with electrode use include irritation, burning and/or dry-out of the skin of the patient.

Accordingly, it is desirable to incorporate a hydrophobic medicament (such as vitamin E or other vitamins having antioxidant properties) into hydrophilic materials of the electrode (e.g., the conductive composition or hydrogel) in order to provide the skin of the patient with a therapeutically effective level of healing or treatment.

It is further desirable to provide a method of forming or constructing an electrode comprising a conductive composition for contact with the skin of a patient that includes a therapeutically effective level of a hydrophobic medicament.

### SUMMARY

The present disclosure relates to biomedical electrodes incorporating hydrophobic material and methods of formation thereof.

According to an aspect of the present disclosure, an electrode for selective attachment to the skin of a subject is provided. The electrode includes a conductive member defining a first side and a second side; a conductive composition disposed on the first side of the conductive member, wherein the conductive composition includes a therapeutically effect quantity of a hydrophobic medicament; and an electrical lead in electrical communication with the conductive member. The hydrophobic medicament of the conductive composition reduces a level of at least one of irritation, damage, dry-out and burning of the skin of the subject.

The hydrophobic medicament may be vitamin E. The conductive composition may include about 10% of vitamin E. The hydrophobic medicament may be vitamin A or fish oil.

The electrode may further include a backing member disposed on the second side of the conductive member. The electrode may further include a release liner selectively, removably adhered to a surface of the conductive composition.

The electrode may further include a reinforcement member supporting the conductive composition. The reinforcement member may be a woven and/or a non-woven cloth or scrim. The reinforcement member may be conductive, for example, by providing a metal or carbon filament therein. The electrode may further include silver (Ag) or silver/silver-chloride (Ag/AgCl) disposed on at least a portion of the first and second sides of the conductive member.

The conductive composition may be a hydrogel. The conductive composition may be a hydrophilic hydrogel and the antioxidant is a hydrophobic vitamin E acetate. The vitamin E acetate may be combined with the hydrogel via a surfactant in an amount so as to prevent the formation of an opaque emulsion.

The electrical lead may be a pig-tail style leadwire, snap style lead or tab style lead.

According to yet another aspect of the present disclosure, a method of forming an electrode is provided. The method includes the steps of providing a conductive member having a first side and a second side; providing a conductive composition on the first side of the conductive member, wherein the conductive composition includes a therapeutically effective quantity of a hydrophobic medicament associated therewith; providing a backing member on the second side of the conductive member; and providing an electrical lead in electrical communication with at least one of the conductive member and the conductive composition.

The hydrophobic medicament may be vitamin E. The conductive composition may include about 0.01-10% of vitamin E.

The method may further include the step of providing silver or silver chloride deposited on at least one of the first side and the second side of the conductive member.

The method may further include the step of providing a release liner on an outer surface of the conductive composition.

The conductive composition may be a hydrogel. The conductive composition may be a hydrophilic hydrogel and the hydrophobic medicament is vitamin E acetate. The method may further include the step of combining the vitamin E acetate with the hydrogel via a surfactant in an amount so as to prevent the formation of an opaque emulsion.

The step of providing a conductive composition may include the steps of providing a quantity of at least one of a hydrophobic material and a hydrophobic medicament; providing a quantity of a surfactant; complexing the surfactant and the hydrophobic medicament; providing a quantity of a hydrophilic material; and combining the complexed surfactant and the hydrophobic medicament with the hydrophilic material.

The method may further include the step of providing a reinforcement member within the conductive composition.

According to a further aspect of the present disclosure, a method of forming a biocompatible conductive composition is provided and includes weighing out a solution of sodium 2-(acrylamido)-2-methylpropanesulfonate (NaAMPS) and placing the NaAMPS is a container; weighing out and adding a cross-linker to the NaAMPS to form a monomer solution; weighing out and adding an acrylic acid to the monomer solution; weighing out and adding a salt to the monomer solution; mixing for approximately 10 minutes; weighing out and adding glycerol to the monomer solution; weighing out and adding silica to the monomer solution; adding sodium hydroxide, drop wise, to the monomer solution to bring the pH of the monomer solution up to about 3.50 ± 0.05; weighing out a quantity of Surfactant and vitamin E acetate to the monomer solution; adding the Surfactant and vitamin E acetate to the monomer solution; weighing out and adding a catalyst to the monomer solution; mixing the monomer solution for approximately 30 minutes; coating the monomer solution on a release liner; and irradiating the coating of the monomer solution under a Xenon arc UV lamp for approximately 25 seconds.

### DETAILED DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a schematic, perspective view of an exemplary electrode of the present disclosure, shown as a leadwire style with layers separated;

FIG. 2 is a cross-sectional view, of the electrode of FIG. 1;

FIG. 3 is a cross-sectional view, of a snap style electrode, according to an embodiment of the present disclosure;

FIG. 4 is a cross-sectional view, of a tab style electrode, according to an embodiment of the present disclosure; and

FIG. 5 is a flow diagram illustrating a method of manufacturing an electrode, according to the principles of the present disclosure.

### DETAILED DESCRIPTION OF EMBODIMENTS

Embodiments of the presently disclosed system and method will now be described in detail with reference to the drawing figures wherein like reference numerals identify similar or identical elements.

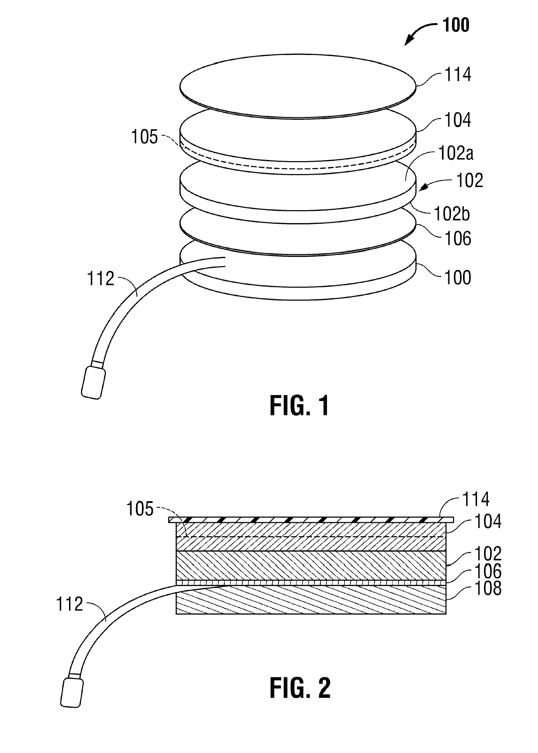

Referring initially to FIGS. 1 and 2, an electrode in accordance with an embodiment of the present disclosure is generally designated as electrode 100. Electrode 100 includes a conductive member 102 defining a first or skin side 102a relative to a subject and a second side 102b, opposite first side 102a. Conductive member 102 may be made from a conductive carbon, aluminum, tin or any other suitable conductive material. As an alternative, conductive member 102 may comprise a conductive plastic material. Conductive member 102 may include silver (Ag) or silver/silver-chloride (Ag/AgCl) material deposited on at least a portion of first side 102a or second side 102b. Either first side 102a or second side 102b may also have a coating of silver (Ag) or silver/silver-chloride (Ag/AgCl) composition or ink 106 on either first side 102a or second side 102b thereof.

Electrode 100 further includes a conductive composition 104 disposed adjacent first side 102a of the conductive member 102 for application/adhesion to or contact with the skin of the subject. Conductive composition 104 may be made from, for example, but not limited to, Promeon RG-63B hydrogel (TycoHealthcare Group LP d/b/a Covidien). As seen in FIGS. 1 and 2, in some embodiments, conductive composition 104 may incorporate a reinforcement member. The reinforcement member may be a woven or non-woven cloth or gauze material (e.g., scrim) 105 embedded therewithin or supporting the structure of the hydrogel. The reinforcement member may be made from a conductive material. The conductive composition 104 may be any different commercially available conductive hydrogel. Conductive composition 104 is generally hydrophilic.

Conductive composition 104 contains a therapeutically effective amount of a hydrophobic medicament incorporated therein. Hydrophobic medicaments may include and are not limited to vitamin E, vitamin A, etc. For example, conductive composition 104 may be provided with vitamin E acetate incorporated therein. Additional hydrophobic medicaments of antioxidants that may be combined with the conductive composition include, and are not limited to, vitamin A, vitamin C, fish oil, or any other composition commonly used to treat skin conditions due to their antioxidant properties. The hydrophobic medicaments is added to conductive composition 104 in an amount sufficient to provide the skin of the subject with a degree of relief from irritation, damage, burning, or drying out of the skin of the patient. The hydrophobic medicament may be provided in the conductive composition 104 at a concentration of about 0.01-10%.

The quantity of hydrophobic material added to the hydrophilic conductive composition 104 is such that an opaque emulsion thereof is avoided. In one exemplary embodiment the conductive composition 104 contains a food grade surfactant (e.g., Antarox® BL236 (aliphatic polyether), commercially available from Rhone Poulenc, Courbevoie, France, at a concentration of about 0.05%), which is used to complex with vitamin E acetate to carry the vitamin E acetate into the hydrogel.

Generally, vitamin E exhibits antioxidant properties that help protect body tissue from damage caused by unstable substances called free radicals. Free radicals can harm cells, tissues, and organs. By providing conductive composition 104 with a therapeutically effective quantity of a hydrophobic medicament such as vitamin E acetate, upon application of electrode 100 to the skin of the subject, the skin of the patient will receive a degree of relief from any irritation, damage, burning, dry-out thereto.

Turning back to FIGS. 1 and 2, a first side release liner 114 is releasably secured to conductive composition 104. Release liner 114 can be made from a film or paper substrate having a release coating on one or both sides, such as, for example silicone. Release liner 114 protects and/or preserves conductive composition 104 (e.g., the hydrogel) and is removed prior to application on the skin of the subject. Release liner 114 may be reapplied to conductive composition 104 after use of electrode 100 to preserve the conductive composition 104 for subsequent use.

Release liner 114 may be a release paper or film of a waxed or coated plastic, such as a silicone coated polyethylene terephthalate film, which may be used to protect electrode 100 before application of the electrode to the skin of the subject.

In an embodiment, electrode 100 may further include a backing member 108 disposed adjacent second side 102b of conductive member 102. In certain embodiments, backing member 108 may overlie silver coating 106. Backing member 108 is fabricated from a non-conductive material such as a cloth, fabric, plastic material or the like.

Electrode 100 further includes an electrical lead, in the form of a lead wire 112 (as shown in FIGS. 1 and 2). Lead wire 112 has a pig tail configuration that is in electrical communication with at least conductive member 102 and a power supply (not shown). An electrical pathway from external equipment to subject skin extends from lead wire 112 through the conductive member 102, and silver coating 106, and through conductive composition 104 to the subject.

In use, release liner 114 is removed from electrode 100. Electrode 100 is then applied to the skin of the subject, such that conductive composition 104 is adhered to the skin of the subject. Electrode 100 is then electrically connected to external medical equipment (not shown) by any connection means well known in the art, such as, for example, via lead wire 112. Electrode 100 may, by way of example, a TENS electrode to be connected to an electrical stimulation device by means known to one having skill in the art.

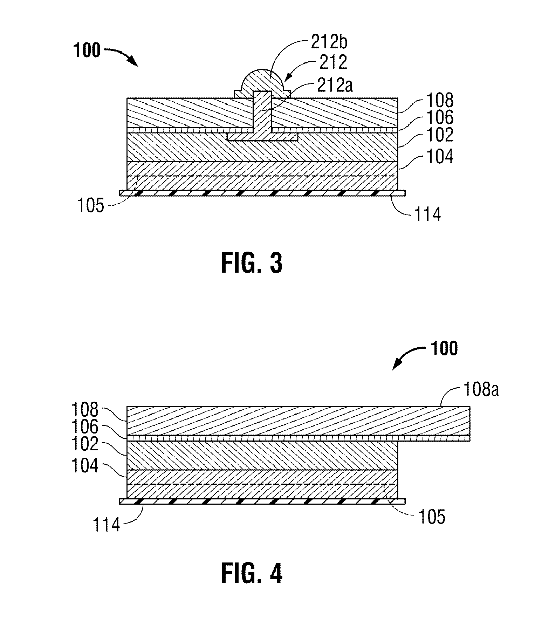

Turning now to FIG. 3, electrode 100 may be configured as a snap-style electrode including an electrical lead, in the form of an electrical snap connector 212. Snap connector 212 includes a post or element 212a in contact with conductive member 102 and extending through silver coating 106 and backing member 108. Snap connector 212 includes a head or stud 212b connected to post 212a.

Referring now to FIG. 4, electrode 100 may be configured as a tab-style electrode wherein backing member 108 includes a portion 108a extending beyond a perimeter or edge of conductive member 102 thereby defining a tab. As seen in FIG. 4, silver coating 106 extends onto the surface of tab portion 108a of backing member 108.

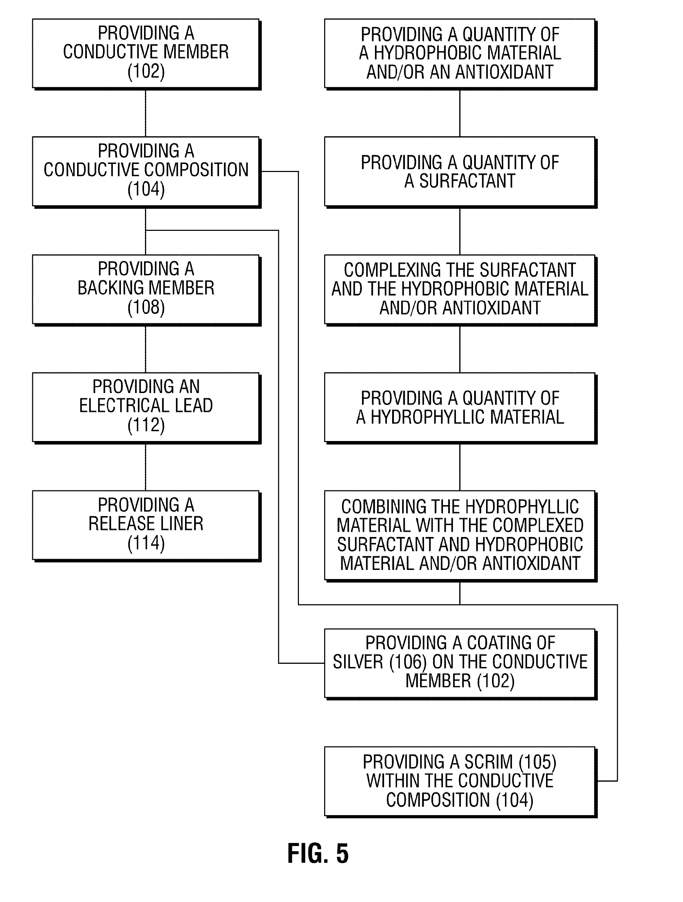

Turning now to FIG. 5, a flow chart of the formation of electrode 100, according to an embodiment of the present disclosure is shown and described. According to the present method, a quantity of a hydrophobic medicament (e.g., vitamin E acetate) is provided. For example, about 1.0 gram of vitamin E acetate may be provided. A quantity of a surfactant (e.g., Antarox® BL236 (aliphatic polyether), commercially available from Rhone Poulenc, Courbevoie, France, at a concentration of about 0.05%) is also provided. For example, about 0.5 grams of surfactant may be provided. It is contemplated that a food grade surfactant be used. The surfactant and vitamin E acetate are then complexed with one another.

A quantity of a hydrophilic material is provided. For example, about 98.95% of a hydrogel may form the basis of the conductive composition. The complexed surfactant and vitamin E acetate are then combined with the hydrogel, in a manner known by one of skill in the art, in such a manner so as to not form an emulsion. It is contemplated that the quantity of vitamin E provided is sufficient to provide a therapeutic effect on the skin of a patient when electrode 100 is applied thereto.

A method of mixing conductive composition 104, according to the principles of the present disclosure includes the following steps, at least some of which are performed sequentially:
1 - weighing out a solution of sodium 2-(acrylamido)-2-methylpropanesulfonate (NaAMPS), approximately 45.416 grams, into a clean container (e.g., a 100ml glass beaker);
2 - placing the container under an electric mixer, wherein the mixer may be equipped with a three blade propeller-style mixing rod;
3 - weighing out a cross-linker, such as, for example, 1% MBA in water solution (approximately 2.980 grams), and adding the cross-linker into the vortex of the NaAMPS located in the mixer forming a monomer solution;
4 - weighing out an acrylic acid, approximately 2.780 grams, and adding the acrylic acid into the vortex of the monomer solution;
5 - weighing out a salt (sodium chloride NaCl), approximately 2.000 grams, and adding the salt into the vortex of the monomer solution;
6 - continuing mixing for approximately 10 minutes;
7 - weighing out a quantity of glycerol, approximately 42.665 grams, and adding the glycerol into the vortex of the monomer solution;
8 - weighing out a quantity of silica, approximately 2.490 grams, and adding the silica into the vortex of the monomer solution;
9 - placing a calibrated pH probe on the edge of the mixing vortex;
10 - adding sodium hydroxide (NaOH 50% solution), drop wise, to the monomer solution to bring the pH of the monomer solution up to 3.50 ± 0.05 (approximately 0.122 grams);
11 - thereafter, removing the pH probe;
12 - weighing out a quantity of Surfactant (Antarox® BI-236 - aliphatic polyether Rhone Poulenc, commercially available from RhonePoulenc, France), approximately 0.050 grams, and vitamin E acetate, approximately 1.000 grams, and adding the Surfactant and vitamin E acetate into the vortex of the monomer solution;
13 - weighing out a quantity of a catalyst (3% catalyst solution), approximately 0.497 grams, and adding the catalyst into the vortex of the monomer solution;
14 - mixing the monomer solution for approximately 30 minutes;
15 - coating the monomer solution at 25mils thick on a release liner;
16 - irradiating the coating of the monomer solution under a Xenon arc UV lamp for approximately 25 seconds; and
17 - covering the resulting polymer film with a release coated polyethylene film, and sealing in a poly-foil pouch.

The vitamin E laced hydrogel may then be used in the construction of an electrode 100 according to methods known in the art.

According to the present disclosure, a method of forming electrode 100 includes, for example, providing a conductive member having a first side and a second side; providing a conductive composition on the first side of the conductive member, wherein the conductive composition includes a therapeutically effective quantity of hydrophobic medicament associated therewith; providing a backing member on the second side of the conductive member; and providing an electrical lead in electrical communication with at least one of the conductive member and the conductive composition.

The antioxidant may be vitamin E. The conductive composition may include about 0.01-10% vitamin E.

The method may further include the step of providing silver (Ag) or silver/silver-chloride (Ag/AgCl) composition or ink on at least a portion of at least one of the first side and the second side of the conductive member.

The method may further include the step of providing a release liner on an outer surface of the conductive composition.

The conductive composition may be a hydrogel. The conductive composition may be a hydrophilic hydrogel and the medicament is a hydrophobic vitamin E acetate. The method may further include the step of combining the vitamin E acetate with the hydrogel via a surfactant in an amount so as to prevent the formation of an emulsion.

The step of providing a conductive composition may include the steps of providing a quantity of at least one of a hydrophobic material; providing a quantity of a surfactant; complexing the surfactant and the hydrophobic material; providing a quantity of a hydrophilic material; and combining the complexed surfactant and the hydrophobic material and an antioxidant with the hydrophilic material.

The method may further include the step of providing a reinforcement member within the conductive composition.

It will be appreciated that various embodiments of the above-disclosure and other features and functions, or alternatives thereof, may be desirably combined into many other different systems or applications. Also that various presently unforeseen or unanticipated alternatives, modifications, variations or improvements therein may be subsequently made by those skilled in the art which are also intended to be encompassed by the following claims. Unless specifically recited in a claim, steps or components of claims should not be implied or imported from the specification or any other claims as to any particular order, number, position, size, or material.

## Claims

1. A biomedical electrode, the electrode comprising:
a conductive member defining a first side and a second side;
a conductive composition disposed on the first side of the conductive member, wherein the conductive composition includes a therapeutically effective quantity of hydrophobic medicament; and
an electrical lead in electrical communication with the conductive member.

2. The electrode according to claim 1, wherein the hydrophobic medicament is vitamin E.

3. The electrode according to claim 2, wherein the conductive composition includes about 0.01-10% vitamin E.

4. The electrode according to claim 2, wherein the conductive composition includes one of greater than about 10% vitamin E, about 0.01-10% vitamin A, and about 0.01-10% fish oil.

5. The electrode according to claim 1, further comprising at least one of a coating of silver and silver-chloride on at least a portion of at least one of the first and second sides of the conductive member.

6. The electrode according to claim 1, wherein the conductive composition is a hydrophilic hydrogel and the hydrophobic medicament is vitamin E acetate, wherein the vitamin E acetate is combined with the hydrogel via a surfactant in an amount so as to prevent the formation of an opaque emulsion.

7. A method of forming an electrode, comprising the steps of:
providing a conductive member having a first side and a second side;
providing a conductive composition on the first side of the conductive member, wherein the conductive composition includes a therapeutically effective quantity of a hydrophobic medicament associated therewith;
providing a backing member on the second side of the conductive member; and
providing an electrical lead in electrical communication with at least one of the conductive member and the conductive composition.

8. The method according to claim 7, wherein the hydrophobic medicament is one of vitamin E, vitamin A and fish oil.

9. The method according to claim 7, wherein the conductive composition includes about 0.01-10% of vitamin E.

10. The method according to claim 7, further comprising the step of providing a coating of silver or silver/silver-chloride on at least a portion of at least one of the first side and the second side of the conductive member.

11. The method according to claim 7, wherein the step of providing a conductive composition includes:
providing a quantity of hydrophobic medicament;
providing a quantity of a surfactant;
complexing the surfactant and the at least one of a hydrophobic material and an antioxidant;
providing a quantity of a hydrophilic material; and
combining the complexed surfactant and the hydrophobic medicament with the hydrophilic material.

12. The method according to claim 7, wherein the conductive composition is a hydrophilic hydrogel and the hydrophobic medicament is vitamin E acetate, and wherein the method further comprises the step of combining the vitamin E acetate with the hydrogel via a surfactant in an amount so as to prevent the formation of an opaque emulsion.

13. The method according to claim 11, wherein the surfactant is aliphatic polyether.

14. A method of forming a biocompatible conductive composition, comprising the step of:
weighing out a solution of sodium 2-(acrylamido)-2-methylpropanesulfonate (NaAMPS) and placing the NaAMPS is a container;
weighing out and adding a cross-linker to the NaAMPS to form a monomer solution;
weighing out and adding an acrylic acid to the monomer solution;
weighing out and adding a salt to the monomer solution;
mixing for approximately 10 minutes;
weighing out and adding glycerol to the monomer solution;
weighing out and adding silica to the monomer solution;
adding sodium hydroxide, drop wise, to the monomer solution to bring the pH of the monomer solution up to about 3.50 ± 0.05;
weighing out a quantity of Surfactant and vitamin E acetate to the monomer solution;
adding the Surfactant and vitamin E acetate to the monomer solution;
weighing out and adding a catalyst to the monomer solution;
mixing the monomer solution for approximately 30 minutes;
coating the monomer solution on a release liner; and
irradiating the coating of the monomer solution under a Xenon arc UV lamp for approximately 25 seconds.

15. The method according to claim 14, further comprising the step of providing at least one of:
approximately 45.416 grams of NaAMPS;
approximately 2.980 grams of a cross-linker;
approximately 2.780 grams of acrylic acid;
approximately 2.000 grams of sodium chloride;
approximately 42.665 grams of glycerol;
approximately 2.490 grams of silica;
approximately 0.122 grams of sodium chloride;
approximately 0.050 grams of surfactant;
approximately 1.000 grams of vitamin E acetate; and
approximately 0.497 grams of a catalyst.

16. The method according to claim 14, further comprising the step of using a pH probe to measure a pH of the monomer probe.
